# EUROPEAN PATENT APPLICATION

(11) **EP 1 862 174 A1**
(43) Date of publication of application: **05.12.2007**
(21) Application number: 06715080.5
(22) Date of filing: 02.03.2006
(51) Int. Cl.: A61K 38/28, A61K 9/08, A61K 47/42, C07K 7/08, C07K 14/00

(54) **INHIBITOR FOR INSULIN POLYMER FORMATION**

(30) Priority: 02.03.2005 JP 2005057911
(71) Applicant: Ajinomoto Co., Inc., Tokyo 104-8315 (JP)
(72) Inventor: SHIMBA, Nobuhisa, -ku, Kawasaki-shi, Kanagawa 2108681 (JP); NAKAMURA, Takefumi, -ku, Kawasaki-shi, Kanagawa 2108681 (JP); SUZUKI, Eiichiro, -ku, Kawasaki-shi, Kanagawa 2108681 (JP)
(74) Representative: HOFFMANN EITLE
(86) International application number: PCT/JP2006/303971
(87) International publication number: WO 2006/093222

(57) **Abstract**

By adding a substance that interacts with the insulin dimer formation interface or the hexamer formation interface to an insulin solution, an insulin preparation showing an ultra-rapid onset of its action, wherein insulin dimer formation and/or hexamer formation are inhibited, is provided.

## Description

### Field of the Invention

The present invention relates to a method of inhibiting the formation of insulin dimers and/or insulin hexamers. The present invention also relates to a substance that inhibits the formation of insulin dimers and/or insulin hexamers, and to an insulin preparation to which this substance is added.

### Brief Description of the Related Art

Diabetes causes chronic systemic metabolic disorders, and the pathogenesis is known to be insulin hyposecretion or insulin resistance. In 2003, the number of world-wide diabetic patients was about two hundred million. However, the number is estimated to exceed three hundred million in 2025. The anti-diabetic drug market reached about 900 billion yen in the world in 2000, and is estimated to exceed two trillion yen by 2006. As described above, the market targeted to diabetes is extremely large, and extensive studies have been made throughout the world.
Anti-diabetic drugs are roughly classified into either insulin preparations or oral preparations, and the insulin preparations account for about 50% of all drug sales. In the past, wild-type insulin was used in insulin preparations, but these preparations require 30 minutes or more after administration to notice the effects. This is due to the property of wild type insulin to form dimers, followed by the formation of hexamers in liquid preparation. It takes a long time for these multimers to dissociate into monomers, which are then easily absorbed by the blood capillaries. Therefore, site-specific mutations were made in wild-type insulin in an attempt to prevent formation of the hexamers. As a result, insulin preparations were developed which demonstrated an ultra-rapid onset of its action, which immediately exhibited the drug's effects. These preparations include Lispro insulin (Eli Lilly and Company), Aspart insulin (Novo Nordisk), and Apidra insulin (Aventis Pharmaceuticals, Inc.). These insulin analogues generally do not form stable hexamers in a preparation, so they are absorbed by the blood capillaries and exhibit the drug's effects immediately after administration. However, there are still various problems due to the fact that these insulin analogues introduced with a site-specific mutation have a different amino acid sequence as compared to wild-type insulin. For example, these insulin analogues easily aggregate compared to the wild-type insulin, and so are not stable (see Patent Document 1 or 2). This is because the hydrophobic interface in the insulin analogue, which is shielded when the dimers or hexamers form, is exposed to the solvent when the analogues are monomers.
As a result, an attempt to develop a more stable insulin analogue is being made. However, when making insulin mutants, unknown side effects or physical properties of such mutants are still feared, even if hexamer formation can be inhibited.
Patent Document 1: Bakaysa, D.L. et al., US 5,474,978
Patent Document 2: Michael, R. et al., US 20030104983

### SUMMARY OF THE INVENTION

The inventors of the present invention started a study for developing an insulin preparation which has an ultra-rapid onset of action using wild-type insulin itself. First, inhibiting dimer and/or hexamer formation without impairing the interaction of insulin to the insulin receptor was attempted. Moreover, they considered that the addition of a substance that inhibits dimer and/or hexamer formation to a wild-type insulin could realize an insulin preparation showing an ultra-rapid onset of its action.
That is, an object of the present invention is to provide a method of inhibiting dimer and/or hexamer formation of wild-type insulin. Another object of the present invention is to provide a substance that inhibits dimer and/or hexamer formation of wild-type insulin and to produce an insulin preparation containing this substance which has an ultra-rapid onset of action.

The inventors of the present invention have made intensive studies for solving the above-mentioned objects. As a result, they have discovered substances capable of inhibiting the formation of insulin dimers and hexamers without impairing the binding activity of insulin to its receptor, and thus completed the present invention.

That is, the present invention includes the followings.
(1) A method of inhibiting the formation of insulin dimers and/or hexamers comprising adding to an insulin solution a substance that interacts with the dimer formation interface or hexamer formation interface of insulin.
(2) The method according to (1), wherein said substance that interacts with the dimer formation interface or hexamer formation interface of insulin is a compound, a peptide, or a protein.
(3) The method according to (1) or (2), wherein said substance that interacts with the dimer formation interface or hexamer formation interface of insulin inhibits dimer and/or hexamer formation without inhibiting the binding of insulin to the insulin receptor.
(4) An agent for promoting insulin monomer formation comprising a substance that interacts with the dimer formation interface or hexamer formation interface of insulin, which is used in the method according to any one of (1) to (3).
(5) A peptide having an activity to inhibit the formation of dimer and/or hexamer of insulin, comprising any one of the following amino acid sequences (a) to (d):
   (a) the amino acid sequence of SEQ ID NO: 4,
   (b) an amino acid sequence of SEQ ID NO: 4 having substitution, deletion, insertion, and/or addition of one or several amino acid residues other than the amino acid residues at positions 1, 4, 5, 8, 9, 12, and 16;
   (c) the amino acid sequence of SEQ ID NO: 1, or 2; and
   (d) an amino acid sequence of SEQ ID NO: 1 or 2 having substitution, deletion, insertion, and/or addition of one or several amino acid residues selected from the amino acid residues at positions 2, 3, 6, 7, 10, 11, 13, 14, and 15.
(6) A peptide that inhibits the formation of dimers and hexamers of insulin comprising the amino acid sequence of SEQ ID NO: 2, in which at least five amino acid residues selected from the group consisting of those at positions 1, 4, 5, 8, 9, 12, and 16 are conserved, and one or several amino acid residues selected from the group consisting of those at positions 2, 3, 6, 7, 10, 11, 13, 14, and 15 is/are substituted, deleted, inserted, and/or added.
(7) A pharmaceutical composition comprising insulin and the peptide according to (5) or (6).
(8) A method of selecting a substance that interacts with the dimer formation interface or hexamer formation interface of insulin and promotes insulin monomer formation comprising:
   (i) contacting candidate substances with an insulin dimer or an insulin hexamer, and measuring insulin monomer formation,
   (ii) contacting the peptide of SEQ ID NO: 2 or an analogue thereof with the insulin dimer or the insulin hexamer to provide a control of insulin monomer formation, and comparing said control with the insulin monomer formation in the step (i), and
   (iii) selecting a substance from the candidate substances in step (i) which has the ability to form insulin monomers that is equal to or higher than the peptide of SEQ ID NO: 2 or the analogue thereof in step (ii).
(9) A method of producing an insulin preparation comprising:
   identifying a substance having the ability to form insulin monomers by the method according to (8), and
   adding the substance to a solution containing insulin.

### BRIEF DESCRIPTION OF THE DRAWINGS

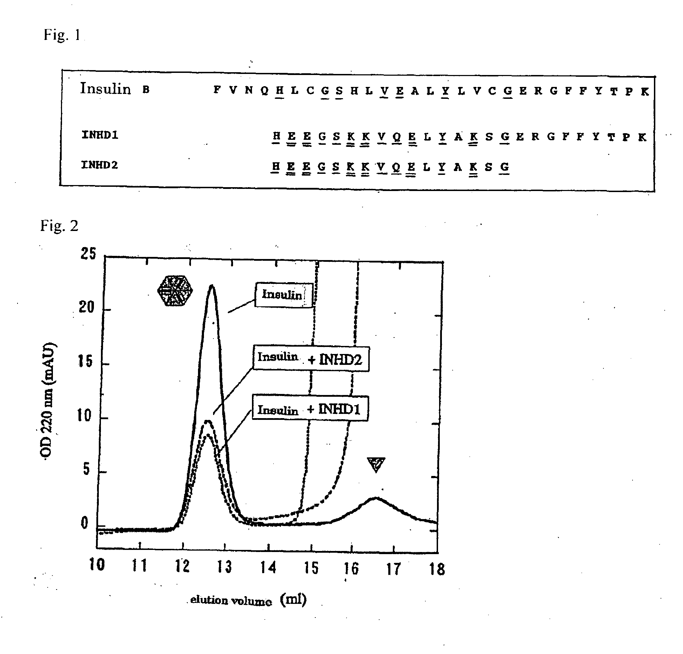

Fig. 1: Sequences of the designed peptides. The single underlines show residues that are present in the insulin dimer interface in the α-helix region of the amino acid sequence of insulin the B-chain. The double underlines show the glutamic acid residues and lysine residues which are introduced so that the α-helix easily forms.
Fig. 2: A gel filtration chromatogram showing the inhibition of insulin hexamer formation by the INHD 1 or INDH2 peptide. The result in the absence of the INHD peptides is also shown as a control (Insulin). Using a 5 µM insulin solution, a hexamer was observed as the main peak (a peak with an elution volume of about 12 to 13 ml), but the insulin hexamer significantly decreased when the INHDI peptide was added.

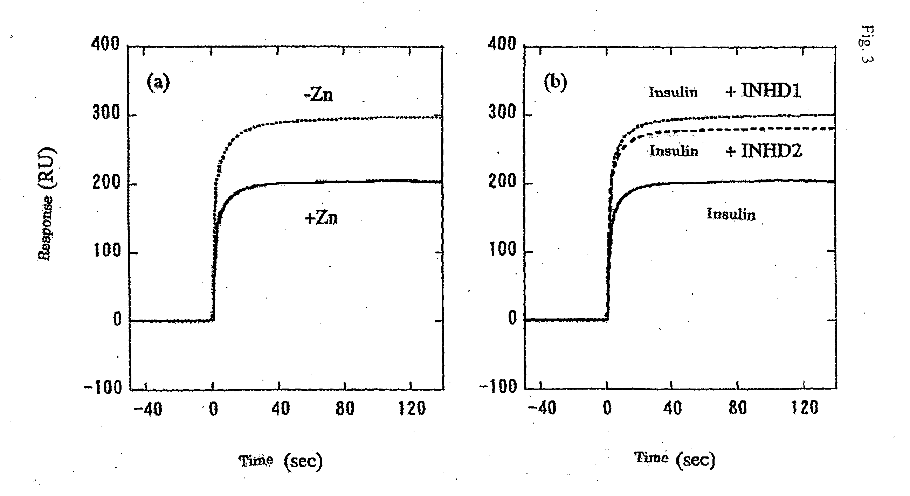

Fig. 3: Analyses of the interaction between insulin and the insulin receptor using BIACORE 2000. An Fc-fusion human insulin receptor was immobilized, and an insulin solution was added at zero time. In all the measurements, the concentration of insulin was 5 µM.
   (a) An experiment on the binding of insulin to the insulin receptor in the presence or absence of ZnCl₂. Insulin forms a hexamer in the presence of ZnCl₂, while monomers are present in the absence of ZnCl₂.
   (b) Binding of insulin to the insulin receptor in the presence or absence of the INHD1 and INHD2 peptides. The solvent contains ZnCl₂, so insulin hexamers form in the absence of the peptides. Addition of the peptides inhibits hexamer formation, resulting in improvement of the interaction between insulin and the insulin receptor.

### DESCRIPTION OF THE PREFERRED EMBODIMENTS

Hereinafter, the present invention will be specifically described.

### <Insulin multimer formation inhibitor>

The substance to be used in the method of inhibiting the formation of insulin dimers and/or insulin hexamers interacts with an insulin dimer formation interface or hexamer formation interface, which results in the inhibition of formation of dimers and hexamers. In the present description, the substance is referred to as an insulin multimer formation inhibitor, an insulin dimer and/or hexamer formation inhibitor, as well as an agent for promoting insulin monomer formation.
The insulin multimer formation inhibitor is preferably a substance selected from low-molecular-weight compounds, peptides, and proteins.

The tertiary structures of insulin dimers and hexamers have been reported, and the interfaces where insulin interacts to form these dimers and hexamers have been clarified (Blundell, T.L. et al., Nature 231, 506-511 (1971), Baker E.N., Philos. Trans. R. Soc. London 319, 369-456 (1998), Derawenda, U. et al., Nature 338, 594-596 (1989), Badger, J. Acta Crystallogr., Sect. B 47, 127-136 (1991)). The insulin dimer formation interface is composed of residues of the α-helix and the subsequent β-strand of the B-chain, and more specifically, the amino acid residues at positions 5, 8, 9, 12, 13, 16, and 20-29 of the B-chain (SEQ ID NO: 3) are involved therein. The insulin hexamer formation interface is composed of the amino acid residues at positions 7, 8, 9, 10, and 13 of the A-chain (SEQ ID NO: 5) and the amino acid residues at positions 3, 7, 10, 11, 14, and 17 of the B-chain. The α-helix of the B-chain is important for the formation of dimers and hexamers, but the interfaces involved in dimer formation are different from those involved in hexamer formation.

The insulin multimer formation inhibitor may be a substance that interacts with an insulin dimer formation interface, an insulin hexamer formation interface, or both. The inhibitor preferably interacts with the dimer formation interface. Prior to formation of a hexamer, insulin first forms a dimer. Specifically, the interaction between the insulin dimer formation interfaces is stronger than the interaction between the insulin hexamer formation interfaces. Therefore, a substance that interacts with the insulin dimer formation interface can effectively inhibit the insulin hexamer formation, as well as dimer formation.

An insulin multimer formation inhibitor may be a substance that also interacts with the insulin hexamer formation interface.

With regard to the interaction between insulin and the insulin receptor, kinetic analysis revealed that two or more insulin molecules bind to the insulin receptor, and that insulin has two binding interfaces (binding interfaces 1 and 2) for the insulin receptor. The binding interfaces of insulin for an insulin receptor have been studied by site-specific mutation, chemical modification, or the like. As a result, it has been found that binding interface 1 includes both the Leu 13 residue of the A-chain and the Leu 17 residue of the B-chain, and that binding interface 2 includes the following residues: A-chain Gly1, A-chain Ile2, A-chain Val3, A-chain Tyrl9, A-chain Asn21, B-chain Val12, B-chain Tyr16, B-chain Gly23, B-chain Phe24, B-chain Phe25, and B-chain Tyr26 (De Meyts, P. et al. Nat. Rev. Drug. Discov. 1, 769-783 (2002)).
Insulin is considered to first interact with the insulin receptor at binding interface 1 (primary binding), and then interact with the insulin receptor at binding interface 2 (secondary binding), resulting in physiological function. Functional insulin requires the interaction with binding interface 2. However, interaction solely at binding interface 2 where a primary binding is not formed results in extremely weak insulin activity, so the primary binding at binding interface 1 is found to be important for efficient functioning of insulin. Meanwhile, it has been revealed that the binding interface 1 plays an important role not only in insulin function but also in the improvement of the affinity of insulin for the insulin receptor and selectivity of the insulin receptor for other receptors, such as the insulin-like growth factor receptor (Schlein, M. et al. Biochemistry 40, 13520-13528 (2001), Pillutla, R.C. et al. J. Biol. Chem. 277, 22590-22594 (2002), Schaffer, L. et al. Proc. Natl. Acad. Sci. USA 100, 4435-4439 (2003)).

Therefore, the insulin multimer formation inhibitor to be used in the present invention is preferably a substance that inhibits insulin dimer formation and hexamer formation and does not significantly impair the interaction between insulin and the insulin receptor. If the substance interacts with a multimer formation interface and a binding interface of insulin to the insulin receptor, it is preferable that the substance interacts with the insulin binding interface 2 with higher affinity than with the insulin binding interface 1. This is because, as described above, efficient insulin function requires that insulin first interacts with the insulin receptor at binding interface 1.
To determine if a substance inhibits multimer formation, the molecular weights can be measured by gel filtration or the like. To determine if the substance inhibits binding to the insulin receptor or not, a commercially available apparatus or kit such as BIACORE can be used to determine the interaction between insulin and the insulin receptor.

The tertiary structure of an insulin dimer includes an α-helix of the insulin B-chain, which interacts with the α-helix of another insulin B-chain. Also, the C-terminal regions interact with each other by forming a β-strand. Therefore, the substance that interacts with the insulin dimer formation interface without interacting with binding interface 1 preferably has a structure of, or similar to, a peptide corresponding to an α-helix and the subsequent C-terminal regions of the insulin B-chain.

### <Method of producing an insulin multimer formation inhibitor>

An insulin multimer formation inhibitor to be used in the present invention may be produced by appropriately selecting for or designing a substance having the above-mentioned properties. In order to design or select for the compound, the ability to form insulin monomers may be tested by screening for an insulin multimer formation inhibitor using a compound designed by the following method of designing an insulin multimer formation inhibitor as a reference compound, thereby performing efficient screening.
(1) Design of insulin multimer formation inhibitor
   As described above, an insulin multimer formation inhibitor of the present invention is preferably a peptide corresponding to an α-heliX and the subsequent C-terminal region of the insulin B-chain, or a substance having a similar structure. To design such a substance, the structure of an appropriate target compound may be designed by obtaining the information on the insulin steric structure from a database such as a protein database (www.rcsb.org/pdb/), and using the molecular coordinate information in the desired region. For example, when designing a compound that mimics functional groups in the dimer interface such as the methyl group of the B-chain Val12, the carboxyamide group of the B-chain Glu13, and the aromatic ring of the B-chain Tyr16, the interatomic distance of each functional group is calculated in the insulin steric structure. To extract low-molecular-weight candidate compounds that have the above functional groups and interatomic distance, a search may be conducted on a compound database such as Available Chemical Database (MDL Information System Inc., San Leandro, USA) using search software such as Sybyl (Tripos Inc., St. Louis, USA) or Isis/Base (MDL Information System Inc., San Leandro, USA). By evaluating inhibition of insulin multimer formation by the obtained candidate compounds by a gel filtration or the like, a low-molecular-weight compound that mimics a dimer interface can be reasonably obtained. When designing the compounds that mimic the dimer interface, the combination of functional groups is not limited to the above-mentioned combination, and it may be a combination of any three or more of side chains of the B-chain His5, Gly8, Ser9, Val12, Glu13, Tyr16, and Gly20, for example. However, the chosen functional groups are not always limited to the above-mentioned amino acid residues.
(2) Design of a peptide having insulin multimer formation inhibitory activity
   When selecting for a peptide corresponding to an α-helix and the subsequent C-terminal region of the insulin B-chain or a peptide having a similar structure, the peptides may be modified so that the α-helix forms more easily so that it can easily interact with the α-helix of the insulin B-chain. For example, in order for the α-helix to form more easily, glutamic acid and lysine residues may be integrated into the peptide at intervals of every three residues. Established methods of producing a peptide that forms an α-helix include a method of cyclizing a peptide with a linker (Judice, J.K. et al., Proc. Natl. Acad. Sci. USA 94, 13426-13430 (1997)) and a method of chelating metal ions (Kelso, M.J. et al., J. Am. Chem. Soc. 122, 10488-10489 (2000)). Also, introducing a required residue into a protein that forms an α-helix as a template (Zondlo, N.J. et al., J. Am. Chem. Soc. 121, 6938-6939 (1999), Schepartz, S.A. et al., U.S. Patent Application 20030166240), or the like has been reported. All of these methods may be used to design a peptide that inhibits insulin dimer and hexamer formation, but the method is not limited thereto.

An example of an amino acid sequence of the insulin B-chain is the amino acid sequence of SEQ ID NO: 3. In this sequence, the residues present in the insulin dimer interface are considered to be important for inhibition of hexamer and/or dimer formation. In the insulin B-chain represented by SEQ ID NO: 3, the amino acid residues at positions 5, 8, 9, 12, 13, 16, and 20 correspond to the dimer interface. Therefore, the peptide having the amino acid sequence from positions 5 to 20 (SEQ ID NO: 4) can be used as an insulin multimer formation inhibitor of the present invention.

Moreover, a peptide having an amino acid sequence obtained by modifying the amino acid sequence of SEQ ID NO: 4, such as the peptides depicted in SEQ ID NO: 1 or 2, may be used. These sequences are obtained by introducing glutamic acid and lysine residues into the amino acid sequence of SEQ ID NO: 4 at intervals of every three residues and replacing the glutamic acid at position 9 with glutamine.
In the peptide of SEQ ID NO: 1 or 2, the amino acid residues at positions 1, 4, 5, 8, 9, 12, and 16 make up the dimer interface.
The residue at position 13 of the insulin B-chain (SEQ ID NO: 3) is glutamic acid, and it is known to be undesirable for hexamer formation due to electrostatic repulsion (Bentley, G.A. et al., J. Mol. Biol. 228, 1163-1176 (1992)). Therefore, in the present invention, the amino acid residue at position 13 of SEQ ID NO: 3 (at position 9 of SEQ ID NO: 4) is replaced by a glutamine residue in the designed peptide (SEQ ID NO: 2) (Fig. 1).
The peptide of SEQ ID NO: 1, 2, or 5, a modified product thereof, and the like may be produced in accordance with general methods of peptide synthesis. Alternatively, they may be produced by genetic recombination using a polynucleotide encoding the peptides. In genetic recombination, the polynucleotide encoding the peptides may be obtained by PCR using primers designed based on the nucleotide sequence encoding insulin (such as the nucleotide sequence registered in GenBank Accession No. J00265). Meanwhile, in the case where the peptides include an amino acid substitution compared to the sequence of a wild-type peptide, a polynucleotide may be modified by site-specific mutation so that the peptides have such a substitution. Furthermore, a polynucleotide encoding the peptide can also be obtained by separately synthesizing a sense strand encoding the peptide and an antisense strand having a sequence complementary to the sense strand, and then annealing the strands. By expressing the thus-obtained polynucleotide in an appropriate host, such as *Escherichia coli,* mammalian cells, or insect cells, the target peptide can then be purified. A polynucleotide may be introduced into host cells by a known method, such as by using a plasmid or a viral vector (Sambrook, J., Fritsch, E.F., and Maniatis, T., "Molecular Cloning A Laboratory Manual, Second Edition", Cold Spring Harbor Laboratory Press (1989)).

In the insulin B-chain (SEQ ID NO: 3), the amino acid residues at positions 5, 8, 9, 12, 13, 16, and 20 (positions 1, 4, 5, 8, 9, 12, and 16 of SEQ ID NO: 4) that form the dimer interface are considered to be important for inhibition of hexamer and dimer formation. Therefore, one or several amino acids other than the above-mentioned residues may be substituted, deleted, inserted and/or added.
Furthermore, one or more amino acids may be added to the sequence of SEQ ID NO: 3 or 4 on the N-terminal side and/or the C-terminal side. If the dimer and/or hexamer formation inhibitors have an excessively large molecular weight, the molecular weight of the complex of the inhibitor and insulin will be large, and it may difficult for the complex to enter the blood vessel. Therefore, the number of additional amino acids is desirably 20 or less on the N-terminal side and/or the C-terminal side in total. In addition, to produce a smaller molecule, the number of additional amino acid residues is more desirably 5 or less on the N-terminal side and/or the C-terminal side in total. The one or two amino acid residues selected from the residues at positions 5, 8, 9, 12, 13, 16, and 20 in the sequence of SEQ ID NO: 3 (positions 1, 4, 5, 8, 9, 12, and 16 in SEQ ID NO: 4), and the residues corresponding to these residues in a modified but similar peptide, may also be substituted with a similar amino acid residue, and the sequence is not limited to the above sequences.
The above-described substitution is preferably a conservative substitution, and examples thereof include a substitution of Ser or Thr for Ala, a substitution of Gln, His, or Lys for Arg, a substitution of Glu, Gln, Lys, His, or Asp for Asn, a substitution of Asn, Glu, or Gln for Asp, a substitution of Ser or Ala for Cys, a substitution of Asn, Glu, Lys, His, Asp, or Arg for Gln, a substitution of Gly, Asn, Gln, Lys, or Asp for Glu, a substitution of Pro for Gly, a substitution of Asn, Lys, Gln, Arg, or Tyr for His, a substitution of Leu, Met, Val, or Phe for Ile, a substitution of Ile, Met, Val, or Phe for Leu, a substitution of Asn, Glu, Gln, His, or Arg for Lys, a substitution of Ile, Leu, Val, or Phe for Met, a substitution of Trp, Tyr, Met, Ile, or Leu for Phe, a substitution of Thr or Ala for Ser, a substitution of Ser or Ala for Thr, a substitution of Phe or Tyr for Trp, a substitution of His, Phe, or Trp for Tyr, and a substitution of Met, Ile, or Leu for Val.

For the peptide (SEQ ID NO: 1 or 2) corresponding to the α-helix of the insulin B-chain, the residues at positions 1, 4, 5, 8, 9, 12, and 16 form the dimer interface, and are considered to be important for inhibition of hexamer and/or dimer formation, and one or several amino acid residues other than the amino acid residues at the above-mentioned positions may be substituted, deleted, inserted, and/or added.
Furthermore, one or more amino acids may be added to the sequence of SEQ ID NO: 1 or 2 on the N-terminal side and/or the C-terminal side. If the dimer and/or hexamer formation inhibitor has an excessively large molecular weight, the molecular weight of the complex of the inhibitor and insulin will be large, and it may be difficult for the complex to enter the blood vessel, and therefore the number of additional amino acid residues is desirably 30 or less on the N-terminal side and/or the C-terminal side in total. In addition, to produce a smaller molecule, the number of additional amino acid residues is more desirably 10 or less on the N-terminal side and/or the C-terminal side in total. Amino acid residues at positions 1, 4, 5, 8, 9, 12, and 16 may also be substituted with similar amino acid residues, and the sequence is not limited to the above sequences. The substitution of the amino acids is preferably a conservative substitution as described above.

### <Method of screening for an insulin multimer formation inhibitor>

The insulin multimer formation inhibitor can also be obtained by a method other than the above-mentioned production method.
That is, screening for an insulin multimer formation inhibitor may be accomplished by:
1) contacting candidate substances with an insulin dimer or hexamer, and measuring monomer formation,
2) contacting the peptide having the sequence of SEQ ID NO: 2 or an analogue thereof with an insulin dimer or hexamer to prepare a control for insulin monomer formation,
3) comparing the test control of step (2) with the insulin monomer formation from step (1), and
4) selecting from the candidates in step (1) a substance which is able to cause formation of insulin monomers which is equal to, preferably 10% or more than that of a peptide having the sequence of SEQ ID NO: 2 or its analogue with substitution, deletion, etc. from the candidate substances.
   Formation of insulin monomers, dimers, and hexamers can be detected by, for example, gel filtration chromatography.
   The interaction of the selected substance with the dimer and/or hexamer formation interface of insulin can be evaluated using a commercially available apparatus or kit such as BIACORE in accordance with the method of Example 4 described below.

The insulin multimer formation inhibitor may only weakly interact with insulin. In this case, the substance is preferably a compound having a dissociation constant for insulin of 0.1 nM or more. This is because the blood concentration of insulin required for physiological function is between 0.1 to 10 nM. A substance having a dissociation constant of 0.1 nM or more does not inhibit insulin-receptor binding regardless of the binding site to insulin. On the other hand, the insulin level in a preparation is very high (for example, 100 U/mL (about 600 µM)), and even a substance that weakly interacts with insulin may inhibit insulin hexamer formation.
Insulin is known to change its structure when it interacts with the insulin receptor via binding interface 1, and this structural change is expected to cause dissociation of the insulin multimer formation inhibitor interacting with an insulin dimer formation interface or hexamer formation interface from insulin.

Examples of wild-type insulin to which the insulin multimer formation inhibitor is applied include, but are not limited to, human insulin, bovine insulin, pig insulin, insulin Lispro, insulin aspart, and Apidra insulin. When administering to human beings, human insulin is preferable from the viewpoint of, for example, reduction of side effects. Insulin may be formulated with protamine, zinc ion, cobalt ion, etc..

If the method of the formation of insulin multimers is applied during the preparation of a general insulin preparation, the formation of insulin dimers and hexamers can be inhibited, resulting in stabilization of the insulin monomers in the preparation. That is, if the insulin multimer formation inhibitor is mixed with insulin, it is possible to produce an insulin preparation with an ultra-rapid onset of action, which acts immediately after subcutaneous administration of insulin. The insulin multimer formation inhibitor of the present invention and insulin may be mixed with a pharmaceutically acceptable carrier, such as a diluent, stabilizer, preservative, or buffer.
The dosage form of the insulin preparation of the present invention is not particularly limited, and examples thereof include an injection, an intranasal agent, a transpulmonary absorption agent, and a percutaneous/transmucosal agent. Also, it is reported that, while direct absorption of insulin in the form of a monomer via the lung, skin, or the like, is preferable, the insulin preparation of the present invention may be in a dosage form other than an injection, showing its versatility. In addition, the preparation can be used for stably storing an insulin monomer. Details on insulin preparations are described in Nipponrinshosha Co., Ltd. Japanese Journal of Clinical Medicine, extra number, "Diabetology for New Era 3", 2002, p179-309, etc., and the insulin preparation with an ultra-rapid onset of action obtained by adding the insulin multimer formation inhibitor can be formulated into a preparation in the same way.
The insulin preparation with ultra-rapid onset of action is useful as a pharmaceutical composition for the prevention or treatment of diabetes. When adding the inhibitor to an insulin solution, the inhibitor is preferably added at a concentration of 0.1 to 10,000-fold with respect to that of insulin.

### Examples

Hereinafter, the present invention will be described more specifically by referring to the following non-limiting examples.

### Example 1: Design of a peptide that inhibits insulin dimer and hexamer formation (Fig. 1)

Insulin hexamer formation occurs when the α-helix and the subsequent C-terminal region of B-chain form a dimer formation interface, and then the electrostatic repulsion by Glul3 of B-chain causes a very weak interaction of hexamer formation interfaces. Therefore, a peptide corresponding to the residues at positions 5 to 29 of the B-chain were partially modified as follows:
(1) glutamic acid and lysine residues were introduced into the peptide at intervals of every three residues so that the peptide can easily form an α-helix. SEQ ID NO: 1 represents the amino acid sequence of this designed peptide, and SEQ ID NO: 3 represents the sequence of the insulin B-chain. In the α-helix region of the insulin B-chain, His5, Gly8, Ser9, Va112, Glul3, Tyr16, and Gly20, (indicated by the single underlines in Fig. 1) are the residues which primarily make up the insulin dimer interface. Therefore, glutamine and lysine residues were introduced at intervals of every three residues at positions other than those described above.
(2) The peptide was modified by replacing the residue corresponding to Glul3 of the B-chain with Gln, to suppress electrostatic repulsion.
(3) The peptide was further modified by replacing the Leu 17 in the B-chain with Ala, which results in very little steric hindrance, and by replacing the Cys19 in the B-chain with Ser so as to avoid oxidation. Hereinafter, this peptide is referred to as Insulin Hexamer Disruptor (INHD1) peptide (SEQ ID NO: 1). In addition, in order to specify an important region in INDH 1 peptide, a smaller peptide corresponding to the insulin B-chain α-helix at positions 5 to 20 was designed and named INDH2 (SEQ ID NO: 2).

### Example 2: Insulin hexamer formation

In order to separately detect insulin hexamers and insulin monomers, gel filtration chromatography was performed. PBS supplemented with 100 µM ZnCl₂ was used as a solvent to form hexamers at a concentration of this experiment (5 µM). This is because zinc ion stabilizes insulin hexamers. The results are shown in Fig. 2. For the 5 µM insulin solution, the hexamer was the main peak, and the ratio of the monomer was extremely low.

### Example 3: Inhibition of insulin hexamer formation by the INDH peptide

The insulin solution was subjected to gel filtration chromatography in the presence of the INDH peptide to show inhibition of insulin hexamer formation by the INDH peptide. Fig. 2 shows the results of an experiment where 5 µM insulin and 500 µM INDH 1 peptide were mixed. In the presence of the INDH1 peptide, the peak which represents the insulin hexamer significantly decreased, and it was found that the INDH1 peptide inhibited insulin hexamer formation.
Moreover, in the case of INHD2 peptide, a similar result was obtained although the inhibition of hexamer formation was slightly lower than that for the INHD1 peptide (Fig. 2).
The results show that the the amino acid residues at positions 1, 4, 5, 8, 9, and 12 in the peptide sequence of SEQ ID NO: 2 are important.

### Example 4: Interaction of insulin and insulin receptor in the presence of the peptides

(1) Construction of Fc-fusion human insulin receptor
   A DNA sequence encoding the Fc-fusion human insulin receptor (Bass, J.J. Biol. Chem. 271, 19367 (1996)) was constructed and inserted into the vector pEF-BOS (Nucleic Acids Res. 1990 September 11; 18(17): 5322). Mammalian cells, FreeStyle 293-F, were cultured at 37°C, and the concentration of the cells was adjusted to 1 x 10⁶ cells/mL. The insulin receptor-expressing plasmid was mixed with Cellfectin (Invitrogen Corporation), and added to the cells, followed by culture for an additional two days. The obtained culture supernatant was purified by protein A affinity chromatography, to yield an Fc-fusion human insulin receptor.
(2) Interaction analysis
   Commercially available insulin (manufactured by Sigma-Aldrich Corp.) and the Fc-fusion human insulin receptor obtained by the method above (item (1)) were used to analyze the interaction between insulin and the insulin receptor (BIACORE 2000, manufactured by Biacore Medical Technologies, Inc.). First, PBS with or without 100 µM ZnCl₂ was used to prepare 5 µM insulin solutions. A gel filtration experiment has revealed that insulin is present mainly as a hexamer in the presence of ZnCl₂ (Fig. 2), and is present mainly as a monomer in the absence of ZnCl₂. The interaction between insulin and the insulin receptor was observed using the respective insulin solutions, and the results showed that the amount of insulin that interacts with the receptor decreased in the presence of ZnCl₂ (Fig. 3a). These results were due to the fact that insulin forms a hexamer in the presence of the zinc ion, resulting in suppression of the interaction with insulin receptor.
   Subsequently, 500 µM INDH1 and INDH2 peptides were added to insulin that forms a hexamer in the presence of ZnCl₂, and the interaction between insulin and the insulin receptor was observed. As a result, it was revealed that, in both the cases, the receptor binding significantly increased to a level approximately equal to that with an insulin monomer (Fig. 3b). It was found that the addition of the INHD1 and INHD2 peptides inhibits insulin hexamer formation.
   Meanwhile, the peptides did not inhibit the binding of insulin to insulin receptor. It was found that receptor binding was not impaired even when hexamer formation was inhibited because insulin has two independent receptor binding sites.

### Industrial Applicability

According to the present invention, it is possible to inhibit insulin dimer formation and insulin hexamer formation without impairing the ability of insulin to bind to the insulin receptor. In addition, it is possible to produce an insulin preparation which has an ultra-rapid onset of action using a wild-type insulin.

## Claims

1. A method of inhibiting the formation of insulin dimers and/or hexamers comprising adding to an insulin solution a substance that interacts with the dimer formation interface or hexamer formation interface of insulin.

2. The method according to claim 1, wherein said substance that interacts with the dimer formation interface or hexamer formation interface of insulin is a compound, a peptide, or a protein.

3. The method according to claim 1 or 2, wherein said substance that interacts with the dimer formation interface or hexamer formation interface of insulin inhibits dimer and/or the hexamer formation without inhibiting the binding of insulin to the insulin receptor.

4. An agent for promoting insulin monomer formation comprising a substance that interacts with a dimer formation interface or hexamer formation interface of insulin, which is used in the method according to any one of claims 1 to 3.

5. A peptide having an activity to inhibit the formation of dimer and/or hexamer of insulin, comprising any one of the following amino acid sequences (a) to (d):
(a) the amino acid sequence of SEQ ID NO: 4;
(b) an amino acid sequence of SEQ ID NO: 4 having substitution, deletion, insertion, and/or addition of one or several amino acid residues other than the amino acid residues at positions 1, 4, 5, 8, 9, 12, and 16;
(c) the amino acid sequence of SEQ ID NO: 1 or 2; and
(d) an amino acid sequence of SEQ ID NO: 1 or 2 having substitution, deletion, insertion, and/or addition of one or several amino acid residues selected from the amino acid residues at positions 2, 3, 6, 7, 10, 11, 13, 14, and 15.

6. A peptide that inhibits the formation of dimer and hexamer of insulin, comprising the amino acid sequence of SEQ ID NO: 2, in which at least five amino acid residues selected from the group consisting of those at positions 1, 4, 5, 8, 9, 12, and 16 in said sequence are conserved, and one or several amino acid residues selected from the group consisting of those at positions 2, 3, 6, 7, 10, 11, 13, 14, and 15 in said sequence is/are substituted, deleted, inserted, and/or added.

7. A pharmaceutical composition comprising insulin and the peptide according to claim 5 or 6.

8. A method of selecting a substance that interacts with the dimer formation interface or hexamer formation interface of insulin and promotes insulin monomer formation comprising the steps of:
(i) contacting candidate substances with an insulin dimer or an insulin hexamer, and measuring insulin monomer formation,
(ii) contacting the peptide of SEQ ID NO: 2 or an analogue thereof with an insulin dimer or a insulin hexamer to provide a control of insulin monomer formation, and comparing the control with the insulin monomer formation in step (i), and
(iii) selecting a substance from the candidate substances in step (i) which has the ability to form insulin monomers as well as, or to a greater extent, than the peptide of SEQ ID NO: 2 or the analogue thereof in step (ii).

9. A method of producing an insulin preparation comprising:
identifying a substance having the ability to form insulin monomers using the method according to claim 8, and
adding the substance to a solution containing insulin.
